# EUROPEAN PATENT SPECIFICATION

(11) **EP 1 638 492 B1**
(45) Date of publication and mention of the grant of the patent: **02.01.2008**
(21) Application number: 04779185.0
(22) Date of filing: 26.07.2004
(51) Int. Cl.: A61K 31/485, A61K 9/70

(54) **PREOPERATIVE TREATMENT OF POST OPERATIVE PAIN**
PRÄOPERATIVE BEHANDLUNG VON POSTOPERATIVEN SCHMERZEN
TRAITEMENT PREOPERATOIRE DE LA DOULEUR POSTOPERATOIRE

(30) Priority: 25.07.2003 US 490363 P
(43) Date of publication of application: 29.03.2006
(73) Proprietor: Euro-Celtique S.A., 2330 Luxembourg (LU)
(72) Inventor: REIDENBERG, Bruce, E., Rye, NY 10580 (US); SPYKER, Daniel, A., Burlingame, CA 94010 (US)
(74) Representative: Ehlich, Eva Susanne
(86) International application number: PCT/US2004/024009
(87) International publication number: WO 2005/011578

(56) References cited:
- WO-A-00/35456
- US-A- 5 968 547
- DATABASE MEDLINE [Online] US NATIONAL LIBRARY OF MEDICINE (NLM), BETHESDA, MD, US; December 2003 (2003-12), ZIMMERMANN M ET AL: "[Postoperative pain therapy in orthopedics]" XP002382747 Database accession no. NLM14655008 & DER ORTHOPÄDE. DEC 2003, vol. 32, no. 12, December 2003 (2003-12), pages 1110-1119, ISSN: 0085-4530
- DATABASE EMBASE [Online] ELSEVIER SCIENCE PUBLISHERS, AMSTERDAM, NL; 1978, DIONNE R A ET AL: "Evaluation of preoperative ibuprofen for postoperative pain after removal of third molars" XP002382748 Database accession no. EMB-1978375678 & ORAL SURGERY ORAL MEDICINE AND ORAL PATHOLOGY 1978 UNITED STATES, vol. 45, no. 6, 1978, pages 851-856,

## Description

This application claims priority to U.S. Serial No. 60/490,363, filed on July 25,2003.

### FIELD OF THE INVENTION

The present invention relates to treatment of postoperative pain. In particular, the present invention relates to methods of ameliorating or eliminating the pain experienced by patients recovering from simple surgical procedures.

### BACKGROUND OF THE INVENTION

Pain can be categorized into three groups: (1) acute pain; (2) continuous pain in terminally ill patients; and (3) other forms of chronic pain. In acute pain, a specific noxious stimulant of limited duration can be identified. Acute pain is often characterized by a distinct onset, usually with identifiable etiology such as trauma or surgery.

Pain resulting from minor non-invasive surgeries is routinely treated by administration of analgesics either at the time of, or after, surgery. Due to the time lapse for analgesics to reach effective blood concentrations and their target effect, the patient may experience pain during this time period.

Over the last decade, methods for the relief of pain following surgery have received much attention. Acute postoperative pain management has evolved to include many other treatment modalities, apart from the traditional method of intramuscular opioids in the postoperative period. Changes in treatment modalities include the introduction of other routes of analgesic administration, new analgesics, and differing methods of producing analgesia.

A recent development in pain control, pre-emptive or pre-operative analgesia, has been gaining support among healthcare professionals (Criado, Lab Anim 2000, 34(3):252-9). In pre-emptive analgesia, analgesics are administered before surgery to enhance the efficacy of these drugs. Reports suggest that giving a local anesthetic before surgery (locally, regionally, by spinal or epidural routes), or the administration of NSAIDs or opiates, results in reduced pain and/or reduced analgesic requirements postoperatively. Preoperative administration of analgesics may prevent or reduce hyperalgesia and inhibit inflammation and pain by reducing the synthesis of prostaglandins in response to surgical injury (Desjardins et al., Anesth Analg 2001, 93(3):721-7). Such treatments may decrease anxiety and relieve preoperative pain, if present.

For example, in a study of dental outpatients undergoing the removal of impacted third molars, pretreatment with a nonsteroidal antiinflammatory drug ibuprofen has shown suppression of postoperative pain when compared to standard therapy without an increase in side effects (Dionne et al., J Clin Pharmacol 1983, 23(1):37-43). The postoperative analgesic efficacy of diclofenac administered preoperatively either as a conventional intramuscular injection or as the available suppository formulation was studied in adult male patients undergoing herniorrhaphy in same day surgery (Pereira et al., Int J Clin Pharmacol Res 1999, 19(2):47-51). This preliminary study demonstrated that both preparations provided analgesia postoperatively, and patients who received the suppository preparation were discharged earlier.

Other conditions for which efficacy of preoperative analgesic in reducing post-operative pain were studied include tonsillectomy in children and appendicectomy in young patients. Ketoprofen was used during tonsillectomy (Kokki et al., Paediatr Anaesth 2002, 12(2):162-7) where as intravenous ketamine was used for appendicectomy (Kakinohana et al., Masui 2000, 49(10):1092-6). Buprenorphine suppository administered preoperatively or intraoperatively was found to be useful for control of postoperative pain (Akatsuka et al., Masui 1996,45(3):298-303).

Buprenorphine is a potent, partial agonist of the µ-opioid receptor that has been shown to be effective to control pain in a wide range of patients when delivered by a number of different routes of administration, including intravenously, epidurally, intrathecally, or sublingually in both young and elderly patients (Inagaki et al., Anesth Analg 1996, 83:530-536; Brema et al., In t J Clin Pharmacol Res 1996, 16:109-116; Capogna et al., Anaesthesia 1988, 43:128-130; Adrianensen et al., Acta Anaesthesiol Belg 1985, 36:33-40; Tauzin-Fin et al., Eur J Anaesthesiol 1998, 15:147-152; Nasar et al., Curr Med Res Opin 1986, 10:251-255). There are several types of' transdermal formulations of buprenorphine reported in the literature. See, for example, U.S. Pat. No. 5,240,711 to Hille et al., U.S. Pat. No. 5,225,199 to Hidaka et al., U.S. Pat. No. 5,069,909 to Sharma et al., U.S. Pat. No. 4,806,341 to Chien et al.; U.S. Pat. No. 5,026,556 to Drust et al.; U.S. Pat. No. 5,613,958 to Kochinke et al.; and U.S. Patent No. 5,968,547 to Reder et al. Transdermal delivery systems of buprenorphine, made by Lohmann Therapie-Systeme GmbH & Co., are currently sold in the European Union under the trademark name TRANSTEC®. These patches contain 20, 30, and 40 mg of buprenorphine, with an approximate delivery or "flux" rate of 35, 52.5, and 70 µg/hr, respectively. Transdermal delivery systems in which fentanyl is the opioid analgesic include, for example, Duragesic. The Duragesic patch purportedly provides adequate analgesia for up to 48 to 72 hours (2 to 3 days).
Oral surgery oral medicine and oral pathology 45(6) 851-856, 1978 describes the use of the NSAID ibuprofen for the pre-operative treatment of post-operative pain.

Despite these advances in the art the complete benefit of preemptive analgesia has not been realized due to limitations in modes of administration of effective doses of analgesics while reducing or eliminating their side effects. Such limitations may lead to less than optimal pain treatment, for example: by providing only short term pain relief; slow acting, insufficient pain relief; opioid blockade; insufficient therapeutic action against inflammation and stiffness; or undesirable biological side effects. Thus, there remains a need for improved methods of treating post-operative pain of patients with preoperative administration of analgesics.

### SUMMARY OF THE INVENTION

The present invention provides a specific dosage regimen of buprenorphine that enables effective analgesia or pain relief for postoperative pain.

Accordingly, the invention provides a method of treating postoperative pain in a patient comprising administering a first buprenorphine-containing dosage form 12 to 96 hours prior to surgery. In another embodiment, a second buprenorphine-containing transdermal dosage form is administered post-operatively, the second dosage form comprising the same or greater dosage form than the first dosage form.

In particular embodiments, the invention further comprises administering a third buprenorphine-containing transdermal dosage form at lease once after the second dosing period.

In a specific embodiment, the method involves a rapid dose escalation of buprenorphine patches to achieve the desired postoperative dosage level comprising (1) administering to the patient a first buprenorphine-containing transdermal dosage form 12 to 96 hours prior to surgery, for a first dosing period that is no longer than 5 days; (2) administering to the patient a second buprenorphine-containing transdermal dosage form for a second dosing period that is no longer than 5 days, wherein the second dosage form comprises the same dosage of buprenorphine as, or a greater dosage of buprenorphine than, the first dosage form; and (3) administering to the patient a third buprenorphine-containing transdermal dosage form for a third dosing period, wherein the third dosage form comprises a greater dosage of buprenorphine than the second dosage form, and the third dosage form is the desired dosage level for the postoperative pain control.

In a preferred embodiment, the method is initiated 4 to 10 days prior to surgery. In another embodiment, the first dosage form is at least 5 mg of buprenorphine. In another embodiment, the second dosage form comprises 10 mg of buprenorphine. In yet a further embodiment, the second dosage form comprises 20 mg of buprenorphine. Still, in other embodiments, the second dosage form comprises 30 or 40 mg of buprenorphine.

The invention also provides a method of treating postoperative pain in a patient undergoing a simple surgery. In one embodiment, the simple surgery may be arthroscopic surgery, excision of a mass, or hernia repair. In other embodiments, the surgery may be spinal fusion, intrathoracic surgery such as coarctation repair, or pelvic surgery such as transabdominal surgery.

The transdermal administration of the invention can be produced by a transdermal system selected from the group consisting of a skin patch, a topical gel, a lotion, an ointment, a transmucosal system, a transmucosal device, and an iontophoretic delivery system.

### DETAILED DESCRIPTION OF THE INVENTION

The present invention provides a method of more quickly achieving effective pain control postoperatively in a patient after a simple surgery, by applying a buprenorphine transdermal patch to a patient prior to surgery. The method of the present invention yields important advantages over prior art dosage regimens for opioids in that it does not increase the incidence of adverse events, for example nausea, while permitting more rapid titration to an effective dose. The method comprises administering to the patient an analgesically effective amount of buprenorphine including administering to the patient a series of transdermal dosage forms with at least one incremental dose of buprenorphine. The invention is based, in part, on the discovery that it is possible to rapidly escalate the dose of transdermal buprenophine from the conventional 7-day period, to achieve effective analgesia without inducing, or at least minimizing, adverse effects. Previous work has shown that a 7-day transdermal buprenorphine dosage form can delay titration to blood levels yielding effective pain therapy, and the application of immediate effective dosages may result in adverse events, especially nausea.

In addition, the concentration of buprenorphine provided by preferred transdermal delivery systems, including, BTDS 5, 10, and/or 20, do not induce an opioid blockade, thereby allowing transdermal delivery of buprenorphine in conjunction with other opioid medications. Thus, the use of transdermal delivery of buprenorphine can be especially advantageous as adjuvant pain therapy in a phase of acute pain, and as a more definitive therapy for the remainder of the post-operative period.

Thus, during the acute pain phase, the patient may be on additional medication to further decrease pain. Such medications include, parenteral, oral or rectal opioids, both mu agonist and partial or mixed agonist/antagonist opioids. As used herein, such opioids include, buprenorphine, morphine, hydromorphone, oxycodon, tramadol, oxymorphone, dihydrocodein, and hydrocodon. In addition, non steroidal anti-inflammatory drugs (NSAmS such as ibuprofen and aspirin) and acetominophen can be given to supplement the opioids. The present invention may be used to supplant existing medications, thereby reducing the need for other types of medication.

Accordingly, the present invention provides a more effective method of administering buprenorphine transdermally, increasing the degree of patient compliance with drug therapy and treatment efficacy. Notably, the reduction in side effects and minimization of complications does not diminish the primary therapeutic effect: pain control. Indeed, the present invention can advantageously achieve increased efficacy of pain control and a decrease in adverse events normally associated with preemptive or preoperative analgesia.

The method comprises administering to the patient an analgesically effective amount of buprenorphine in a dosage regimen including administering to the patient a single transdermal dosage form. The transdermal dosage regimen of the invention may be a 5, 10, 20, 30, or 40 mg buprenorphine transdermal patch.

The dosage regimen of the present invention may alternatively be described in terms of administration of a series of transdermal dosage forms comprising administering at least one dosage of buprenorphine 24-96 hours prior to simple surgery. This treatment regime refers to the application of the transdermal dosage forms at minimum 12 hours prior to surgery, and possibly up to seven days maximum, if the patient is already being treated for pain. Further, after 2-7 days of wearing the initial patch, it is removed and replaced with another patch of the same or greater dosage if postoperative pain is not under control.

As used herein, "BTDS" means "Buprenorphine Transdermal System", and "BTDS X", wherein "X" is a number higher than zero, means a transdermal dosage form containing X milligrams of buprenorphine. Thus, "BTDS 5" contains 5 mg buprenorphine. As discussed below, the invention provides kits containing the desired dosage series. Preferably, a BTDS contains buprenorphine in the form of a base or a salt, more preferably in the form of a base.

The method of the present invention may be administered to any patient in need of postoperative pain treatment. The patient may be classified, but need not be, a patient undergoing simple surgery. As used herein, a "simple surgery" refers to those surgeries not requiring entrance into a major body cavity. The simple surgeries include but are not limited to hernia repair, excision of masses, and arthroscopic surgery.

As used herein, the term "predefined number of days" refers to a predetermined length of time during which the dosage form of the drug is administered to the patient. Preferably, the drug is an opioid and more preferably the opioid is buprenorphine. The predefined number of days may vary between individuals and may be determined by one of ordinary skill in the art using the guidelines discussed within the present application. In a further embodiment, the predefined number of days is 1-3 days.

The term "adverse event" (AE) or "adverse experience" herein means any untoward medical occurrence in a patient or clinical investigation subject administered a pharmaceutical product and which does not necessarily have to have a causal relationship with this treatment A serious adverse event (experience) or reaction is any medical occurrence that at any dose: results in death, is life-threatening, requires inpatient hospitalization or prolongation of existing hospitalization, results in persistent or significant disability/incapacity, or is a congenital anomaly/birth defect. (Guideline for Industry - Clinical Safety Data Management: Definitions and Standards for Expedited reporting. ICH-E2A, March 1995. World Wide Web (www.) address fda.gov/MedWatch/report/iche2a.pdf, pp. 5-7). Exemplary adverse events in a treatment regimen include, but are not limited to, respiratory depression, choleocystitis, abdominal pain, dizziness, orthostatic hypotension, and nausea.

An "analgesically effective" amount of an analgesic agent means an amount of agent capable of lowering the level of pain experienced by a patient. The level of pain experienced by a patient can be assessed by use of a visual analog scale (VAS) or a Likert-type scale. A VAS is a straight line with one end of the line representing no pain and the other end of the line representing the worst imaginable pain. Patients are asked to mark on the line where they considered their pain to be at each time point, and the length from no pain to the mark can be related to the length of the full scale. A Likert-type scale is a rating scale, usually in the range of 1 to 5, based on degrees of agreement or disagreement to statements. A similar type of scale, although based on an 11 point scale (ranging from 0 to 10) can also be used. Such pain scales can be applied to visualize an alteration of the level of pain a patient experiences during treatment, e.g., a reduction of the level of pain a patient or a population of patients experiences before and after initiation of a pain therapy.

### Buprenorphine

The present invention relates to buprenorphine or a pharmaceutically acceptable salt, ether derivative, ester derivative, acid derivative, enantiomer, diasteriomer, racemate, polymorph, or solvate thereof. Pharmacologically, without being bound to any particular theory, buprenorphine is considered in the art to be a partial agonist at µ opioid receptors in the central nervous system ("CNS") and peripheral tissues. Buprenorphine shares many of its actions, such as analgesia, of full µ opioid agonists. Partial agonists, generally, include compounds with affinity for a receptor, but unlike full agonists, elicit only a small degree of the pharmacological effect, even if a high proportion of receptors are occupied by the compound. A "ceiling effect" to analgesia *(i.e.,* no additional analgesia with increasing dose) is well documented with respect to buprenorphine in many animal models. It is highly lipophilic and dissociates slowly from opioid receptors. It is further thought that buprenorphine binds with high affinity to µ and κ₁ receptors, and, with lower affinity, to δ receptors. The intrinsic agonist activity at the κ receptor seems to be limited and most evidence suggests that buprenorphine has antagonist activity at κ receptors. The lack of κ agonism accounts for buprenorphine's freedom from the dysphoric and psychotomimetic effects often seen with agonist/antagonist drugs. Other studies suggest that the opioid antagonist effects of buprenorphine may be mediated via an interaction with δ opioid receptors.

It is known in the art that buprenorphine binds slowly with, and dissociates slowly from, the µ receptor. The high affinity of buprenorphine for the µ receptor and its slow binding to, and dissociation from, the receptor is thought to possibly account for the prolonged duration of analgesia and, in part, for the limited physical dependence potential observed with the drug. The high affinity binding may also account for the fact that buprenorphine can block the µ agonist effects of other administered opioids.

Like other opioid agonists, buprenorphine produces dose-related analgesia. The exact mechanism has not been fully explained, but analgesia appears to result from a high affinity of buprenorphine for µ and possibly κ opioid receptors in the central nervous system. The drug may also alter the pain threshold (threshold of afferent nerve endings to noxious stimuli). On a weight basis, the analgesic potency of parenteral buprenorphine appears to be about 25 to about 50 times that of parenteral morphine, about 200 times that of pentazocine, and about 600 times that of meperidine.

### Salts and Derivatives

Use of various pharmaceutically acceptable salts, ether derivatives, ester derivatives, acid derivatives, and aqueous solubility altering derivatives of the active compound also are encompassed by the present invention. The present invention further includes the use of all active individual enantiomers; diastereomers, racemates, and other isomers of the compound. The invention also includes the use of all polymorphs and solvates, such as hydrates and those formed with organic solvents, of this compound. Such isomers, polymorphs, and solvates may be prepared by methods known in the art, such as by regiospecific and/or enantioselective synthesis and resolution.

Suitable salts of the compound include, acid addition salts, such as those made with hydrochloric, hydrobromic, hydroiodic, perchloric, sulfuric, nitric, phosphoric, acetic, propionic, glycolic, lactic pyruvic, malonic, succinic, maleic, fumaric, malic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin; alkali metal salts, such as sodium and potassium salts; alkaline earth metal salts, such as calcium and magnesium salts; and salts formed with organic or inorganic ligands, such as quaternary ammonium salts.

Additional suitable salts include, but are not limited to, acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate salts of the compound.

The present invention includes prodrugs of the compound. Prodrugs include, but are not limited to, functional derivatives of buprenorphine that are readily convertible *in vivo* into buprenorphine. Conventional procedures for the selection and preparation of suitable prodrug derivatives are described, for example, in "Design of Prodrugs", ed. H. Bundgaard, Elsevier, 1985.

### Transdermal Dosage Forms

Transdermal dosage forms are convenient dosage forms for delivering many different active therapeutically effective agents, including but not limited to analgesics, such as for example, opioid analgesics. Typical opioid analgesics include, but are not limited to, fentanyl, buprenorphine, etorphines, and other high potency narcotics. Transdermal dosage forms are particularly useful for timed release and sustained release of active agents.

Transdermal dosage forms may be classified into transdermal dosage articles and transdermal dosage compositions. The most common transdermal dosage article is a diffusion-driven transdermal system (transdermal patch) using either a fluid reservoir or a drug-in-adhesive matrix system. Transdermal dosage compositions include, but are not limited to, topical gels, lotions, ointments, transmucosal systems and devices, and iontophoretic (electrical diffusion) delivery systems. Preferably, the transdermal dosage form is a transdermal patch.

Transdermal patch dosage forms used in accordance with the invention preferably include a backing layer made of a pharmaceutically acceptable material which is impermeable to the buprenorphine. The backing layer preferably serves as a protective cover for the buprenorphine, and may also provide a support function. Examples of materials suitable for making the backing layer are films of high and low density polyethylene, polypropylene, polyvinylchloride, polyurethane, polyesters such as poly(ethylene phthalate), metal foils, metal foil laminates of such suitable polymer films, textile fabrics, if the components of the reservoir cannot penetrate the fabric due to their physical properties, and the like. Preferably, the materials used for the backing layer are laminates of such polymer films with a metal foil such as aluminum foil. The backing layer can be any appropriate thickness to provide the desired protective and support functions. A suitable thickness will be from about 10 to about 200 microns. Desirable materials and thickness will be apparent to the skilled artisan.

In further embodiments, the transdermal dosage forms used in accordance with the invention contain a biologically acceptable polymer matrix layer. Generally, the polymers used to form the polymer matrix are those capable of forming thin walls or coatings through which pharmaceuticals can pass at a controlled rate. A non-limiting list of exemplary materials for inclusion in the polymer matrix includes polyethylene, polypropylene, ethylene/propylene copolymers, ethylene/ethylacrylate copolymers, ethylenevinyl acetate copolymers, silicones, rubber, rubber-like synthetic homo-, co- or block polymers, polyacrylic esters and the copolymers thereof, polyurethanes, polyisobutylene, chlorinated polyethylene, polyvinylchloride, vinyl chloride-vinyl acetate copolymer, polymethacrylate polymer (hydrogel), polyvinylidene chloride, poly(ethylene terephthalate), ethylene-vinyl alcohol copolymer, ethylene-vinyloxyethanol copolymer, silicones including silicone copolymers such as polysiloxane-polymethacrylate copolymers, cellulose polymers (e.g., ethyl cellulose, and cellulose esters), polycarbonates, polytetrafluoroethylene and mixtures thereof. Exemplary materials for inclusion in the polymer matrix layer are silicone elastomers of the general polydimethylsiloxane structures, (e.g., silicone polymers). Preferred silicone polymers cross-link and are pharmaceutically or biologically acceptable. Other preferred materials for inclusion in the polymer matrix layer include: silicone polymers that are cross-linkable copolymers having dimethyl and/or dimethylvinyl siloxane units that can be crosslinked using a suitable peroxide catalyst. Also preferred are those polymers consisting of block copolymers based on styrene and 1,3-dienes (particularly linear styreneisoprene-block copolymers of styrene-butadiene-block copolymers), polyisobutylenes, polymers based on acrylate and/or methacrylate.

The polymer matrix layer may optionally include a pharmaceutically acceptable crosslinking agent. Suitable crosslinking agents include, e.g., tetrapropoxy silane. Preferred transdermal delivery systems used in accordance with the methods of the present invention include an adhesive layer to affix the dosage form to the skin of the patient for the desired period of administration. If the adhesive layer of the dosage form fails to provide adhesion for the desired period of time, it is possible to maintain contact between the dosage form with the skin by, for instance, affixing the dosage form to the skin of the patient with an adhesive tape, e.g., surgical tape.

The adhesive layer preferably includes using any adhesive known in the art that is pharmaceutically compatible with the dosage form and preferably hypoallergenic, such as polyacrylic adhesive polymers, acrylate copolymers (e.g., polyacrylate) and polyisobutylene adhesive polymers. In alternative embodiments of the invention, the adhesive is a hypoallergenic and pressure-sensitive contact adhesive.

The transdermal dosage forms that can be used in accordance with the present invention may optionally include a permeation enhancing agent. Permeation enhancing agents are compounds which promote penetration and/or absorption of the buprenorphine through the skin or mucosa and into the blood stream of the patient. A non-limiting list of permeation enhancing agents includes polyethylene glycols, surfactants, and the like.

Alternatively, permeation of buprenorphine may be enhanced by occlusion of the dosage form after application to the desired site on the patient with, e.g. an occlusive bandage. Permeation may also be enhanced by removing hair from the application site by, e.g. clipping, shaving or use of a depilatory agent. Another permeation enhancer is heat. It is thought that permeation can be enhanced by, among other things, using a radiating heat form, such as an infrared lamp, at the application site during at least a portion of the time the transdermal dosage form is applied on the skin or mucosa. Other means of enhancing permeation of buprenorphine, such as the use of iontophoretic means, are also contemplated to be within the scope of the present invention.

A preferred transdermal dosage form that may be used in accordance with the present invention includes a non-permeable backing layer made, for example, of polyester; an adhesive layer made, for example, of a polyacrylate, and a matrix containing the buprenorphine and other desirable pharmaceutical aids such as softeners, permeability enhancers, viscosity agents and the like.

The active agent, buprenorphine, may be included in the device in a drug reservoir, drug matrix or drug/adhesive layer. This area of the patch, and the amount of active agent per unit area, determine the limit dose, as one of ordinary skill in the art can readily determine.

Certain preferred transdermal delivery systems also include a softening agent in the reservoir or matrix. Suitable softening agents include higher alcohols such as dodecanol, undecanol, octanol, esters of carboxylic acids, wherein the alcohol component may also be a polyethoxylated alcohol, diesters of dicarboxylic acids, such as di-n-butyladiapate, and triglycerides, particularly medium-chain triglycerides of the caprylic/capric acids or coconut oil. Further examples of suitable softeners are multivalent alcohols, for example, levulinic acid, coprylic acids, glycerol and 1,2-propanediol which can also be etherified by polyethylene glycols.

A buprenorphine solvent may also be included in the transdermal delivery systems of the present invention. Preferably, a solvent dissolves the buprenorphine to a sufficient extent thereby avoiding complete salt formation. A list of suitable solvents include those with at least one acidic group. Particularly suitable are monoesters of dicarboxylic acids such as monomethylglutarate and monomethyladipate.

Other pharmaceutically acceptable components that may be included in the reservoir or matrix include solvents, for example, alcohols such as isopropanol; permeation enhancing agents such as those described above; and viscosity agents, such as cellulose derivatives, natural or synthetic gums, such as guar gum, and the like.

In alternative embodiments, the transdermal dosage form includes a removable protective or protective release layer. The removable protective layer is removed prior to application; and may consist of the material used for the backing layer described above, provided that it is rendered removable, for example, by a silicone treatment. Other removable protective layers, for example, are polyletra-fluoroethylene, treated paper, allophane, polyvinyl chloride, and the like. Generally, the removable protective layer is in contact with the adhesive layer and provides a convenient means of maintaining the integrity of the adhesive layer until the desired time of application.

The composition of the transdermal dosage form used in accordance with the invention and the type of device used are not considered critical to the method of the invention, provided that the device delivers the active agent, e.g. buprenorphine, for the desired time period and at the desired flux rate and/or the desired delivery rate, *i.e.,* the rate of penetration of the active agent through the skin of an individual, of the transdermal dosage form.

Certain preferred transdermal dosage forms for use in accordance with the present invention are described in U.S. Pat. No. 5,240,711 to Hille, et. al.; (assigned to LTS Lohmann Therapie-Systeme GmbH & Co.) Such buprenorphine transdermal delivery systems may be a laminated composite having an impermeable backing layer containing buprenorphine, and optionally, a permeation enhancer combined with a pressure-sensitive adhesive. A preferred transdermal dosage form in accordance with the 5,240,711 patent includes: (i) a polyester backing layer which is impermeable to buprenorphine; (ii) a polyacrylate adhesive layer; (iii) a separating polyester layer; and (iv) a matrix containing buprenorphine or a salt thereof, a solvent for the buprenorphine, a softener and a polyacrylate adhesive. The buprenorphine solvent may or may not be present in the final formulation. The transdermal delivery device described therein includes a backing layer which is impermeable to the active substance, a pressure-sensitive adhesive reservoir layer and optionally, a removable protective layer. Preferably, the matrix includes about 10 to about 95% (by weight) polymeric .material, about 0.1 to about 40% (by weight) softener, and about 0.1 to about 30% (by weight) buprenorphine. A solvent for the buprenorphine base or pharmaceutically acceptable salt thereof may be included as about 0.1 to about 30% (by weight).

The dosage forms of the present invention may also include one or more inactivating agents. The term "inactivating agent" refers to a compound that inactivates or crosslinks the active agent, in order to decrease the abuse potential of the transdermal dosage form. Non-limiting examples of inactivating agents include, but are not limited to, polymerizing agents, photo-initiators, and formalin. Examples of crosslinking or polymerizing agents include diisocyanates, peroxides, diimides, diols, triols, epoxides, cyanoacrylates, and UV activated monomers.

Any appropriate additives, inactivating agents, and dosage forms that are known in the art may be used in combination with the method of the invention.

Topical preparations typically contain a suspending agent and optionally, an antifoaming agent. Such topical preparations may be liquid drenches, alcoholic solutions, topical cleansers, cleansing creams, skin gels, skin lotions, and shampoos in cream or gel formulations (including, aqueous solutions and suspensions).

Alternatively, buprenorphine can be administered in the form of a liposome delivery system. For example, small unilamellar vesicles, large unilamellar vesicles and/or multilamellar vesicles may be included in the transdermal article or transdermal composition. Liposomes can be formed from a variety of phospholipids, such as cholesterol, stearylamine or phosphatidylcholines.

The transdermal dosage form may be formulated by any method known in the art and may be administered as suggested. Such formulations are described in U.S. Patents 4,806,341; 5,240,711; and 5,968,547.

### Administration

The unit dosage forms of the present invention are administered to a patient, preferably a human patient, suffering from postoperative pain. In an alternative embodiment, the patient is scheduled to undergo a simple surgery. The unit dosage forms of the present invention may be administered at the defined dosing regimen in order to rapidly achieve optimal activity while reducing any potential toxicity. For example, the method involves administering to the patient an analgesic effective amount of buprenorphine in a dosage regimen comprising a series of transdermal dosage forms of graduated and ascending dosages of buprenorphine. Preferably, the dosage regimen comprises the steps of:
(a) a first buprenorphine-containing transdermal dosage form for a first dosing period prior to simple surgery; and
(b) a second buprenorphine-containing transdermal dosage form for a second dosing period, wherein the second dosage form comprises the same or a greater dosage of buprenorphine than the first dosage form after surgery.

In a further embodiment, a third buprenorphine-containing transdermal dosage form may be administered for a third dosing period after the operation, wherein the third dosage form comprises the same greater dosage of buprenorphine than the second dosage form.

In another embodiment, the invention may be used in more severely traumatic surgeries such as extensive orthopedic surgery including but not limited to spinal fusion, intrathoracic surgery (i.e., coarctation repair), or pelvic surgery (i.e., transabdominal hysterectormy).

The dosing regimen of the present invention comprises dosing periods prior to surgery. A dosing period is the time during which one of the transdermal dosage forms in the series is administered to the patient, and the dosing regimen will consist of a separate dosing period for administration of each transdermal dosage form in the series. Thus, for example, the transdermal dosage form in the series may be worn by the patient for at least one, or, in another embodiment, three consecutive days prior to surgery. The patch may, for example, be place at the mid-axillary line at the fifth intercostal space. Upon removal, a second dosage form may then be worn by the patient for another 3-7 consecutive days, and thereafter, a third dosage form may be worn by the patient for another seven days if pain persists. In a further embodiment, the total treatment period of the dosing regimen is 7 days, including the preoperative days. This dose can then be maintained indefinitely. If an increase in dosage is required, then the dosage may be increased at an appropriate interval, e.g., every 3-7 days.

In a specific embodiment the first dosage form comprises up to 5 mg buprenorphine, and the first dosing period is at least 3 days; the second dosage form comprises up to 10 mg buprenorphine, and the second dosing period is at least 3 days; and the third dosage form comprises up to 20 mg buprenorphine, and the third dosing period is 2-3 days.

In another embodiment, subsequent dosages may be administered. For example, if the target analgesia level is attained with the third dosing period, fresh replacements of the third dosage form can be repeatedly administered for an extended period of time, changing patches with a frequency extending from about every 2 days to weekly. If the target analgesia level is not attained with the third dosing period, subsequent dosage forms, comprising incrementally increasing levels of buprenorphine, can be applied, starting with 30 mg buprenorphine and 40 mg buprenorphine load.

The method of the present invention preferably administers buprenorphine in a way that achieves a rapid increase in the plasma concentration of buprenorphine in the patient, more preferably still without inducing nausea or other adverse events. In a further embodiment, the plasma profile achieved by the method of the present invention may be described as follows, the mean plasma buprenorphine concentration at the commencement of surgery between 400-2000 pg/ml (2 ng/ml)or higher depending on the patient's need.

The dosage of buprenorphine may vary according to a variety of factors such as underlying disease states, the individual's condition, weight, sex and age and the mode of administration. The dosage predefined interval or regimen is selected in accordance with a variety of factors including species, age, weight, sex and medical condition of the patient; the severity of the condition to be treated; the selected transdermal delivery system; the renal and hepatic function of the patient; and the particular form of buprenorphine used. A physician or veterinarian of ordinary skill will readily be able to determine and prescribe the effective amount of the drug required to prevent, counter or arrest the progress of the condition in view of this disclosure. Optimal precision in achieving concentrations of drug within the range that yields efficacy without toxicity requires a regimen based on the kinetics of the drug's availability to target sites. This involves a consideration of the absorption, distribution, metabolism, and excretion of a drug.

The composition or dosage form of the invention, when administered as a transdermal dosage form, may be provided to any body part as determined by one of ordinary skill in the art. For example, the composition or dosage form may be provided to the arm, leg or chest of the patient. In the preferred embodiment for children, the placement is preferably on the back to prevent the removal of the transdermal unit. Repeated doses may or may not be administered to the same location each time.

Generally, topical preparations contain from about 0.01 to about 100% by weight and preferably from about 3 to about 80% by weight of the compound, based upon 100% total weight of the topical preparation. Generally, transdermal dosage forms contain from about 0.01 to about 100% by weight and preferably from about 3 to about 50% by weight of the compound, based upon 100% total weight of the buprenorphine formulation in the dosage form.

The dosage forms used in the method of the present invention may be administered alone or in combination with other active agents. For combination treatment with more than one active agent, where the active agents are in separate dosage formulations, the active agents can be administered concurrently, or they each can be administered at separately staggered times. The dosage amount may be adjusted when combined with other active agents as described above to achieve desired effects. Alternatively, unit dosage forms of these various active agents may be independently optimized and combined to achieve a synergistic result wherein the pathology is reduced more than it would be if either active agent were used alone.

In an exemplary embodiment, at the pre-anesthetic visit, the night before the operation, the anesthesiologist assesses the patient and considers BTDS appropriate care. The patient receives BTDS 5 that night. The following day, wearing BTDS 5, the patient goes to the Operating Room and has a successful procedure under balanced anesthesia. Postoperatively, the patient is managed with patient controlled intravenous mu-agonist opiates. If the patient requires more than 2 rescue opiate doses per day, the BTDS is increased on an every 3-day basis. When the patient is capable of taking oral medication, the patient is given nonsteroidal antiinflammatory drugs (NSAID such as ibuprofen) and short acting oral mu-agonist opiates (such as oxycodone) in addition to the BTDS still on the patient. When the patient no longer requires rescue medication and the physician feels healing is accomplished, such that pain is not anticipated, the BTDS is removed.

### Kits

The present invention also provides an embodiment wherein the components for practicing the invention can be conveniently provided in a kit form. In its simplest embodiment, a kit of the invention provides a set number of buprenorphine patches at set dosages, wherein the dosages are set according to the needs of the patient. Each kit will include a starter kit with instructions for application and the appropriate dosage regimen selected from the following: BTDS 5, 10, 20, 30, or 40 mg.

In a further embodiment, the kit will contain a dosage regimen for rapid dosage escalation to reach the desired preoperative dosage level. Again, the dosage escalation will include appropriate dosage regimen selected from the following: BTDS 5, 10, 20, 30, or 40 mg. Printed instructions on how to apply the patch, storage of the unit, and details of the treatment regimen are also included.

A kit of the invention preferably includes packaging and printed instructions for its use, e.g., on the packaging or package insert, including when to initiate prophylactic treatment, and when to switch to high dose patches or dosage forms. The buprenorphine patches within the kit may be coded (i.e., color, numerical by day, or numerical by dose, etc.) for the patient. For example, the printed instructions may describe the use of the dosage regiment to treat or prevent diarrhea or other gastrointestinal conditions or disorders.

In a further embodiment, the kit will include a disposal container or device for disposal of used buprenorphine patches. Any such containers or devices can be used to prevent or limit potential abuse of the drug within the patch. As used herein, the term container has its broadest meaning, *i.e.,* any receptacle for holding material.

## Claims

1. Use of buprenorphine for the manufacture of a transdermal dosage form for treating postoperative pain in a patient in need of such treatment, the treatment comprising administering to said patient said buprenorphine-containing transdermal dosage form prior to surgery.

2. The use of claim 1, wherein the treatment comprises applying the buprenorphine-containing transdermal dosage form 12 - 96 hours prior to surgery.

3. The use of claim 1, wherein the treatment further comprises the administration of a second buprenorphine-containing transdermal dosage form for a second dosing period postoperatively, wherein said second dosage form comprises the same dosage of buprenorphine as, or a greater dosage of buprenorphine than, said first dosage form.

4. The use of claim 1, wherein the treatment further comprises extended subsequent dosing periods with subsequent dosage forms for a given time period as needed by the patient to achieve desired analgesia.

5. The use of claim 1, wherein the treatment further comprises a rapid dose escalation of buprenorphine patches to achieve the desired preoperative dosage level, wherein the method involves:
(a) administering to the patient a first buprenorphine-containing transdermal dosage form for a first dosing period that is no longer than 5 days;
(b) administering to the patient a second buprenorphine-containing transdermal dosage form for a second dosing period that is no longer than 5 days, wherein the second dosage form comprises the same dosage of buprenorphine as, or a greater dosage of buprenorphine than, the first dosage form; and
(c) administering to the patient a third buprenorphine-containing transdermal dosage form for a third dosing period, wherein the third dosage form comprises a greater dosage of buprenorphine than the second dosage form, and the third dosage form is the desired dosage level for the postoperative pain control.

6. The use of claim 5, wherein the treatment is initiated 4-10 days prior to surgery.

7. The use of claim 1, wherein said dosage form comprises at least 5 mg of buprenorphine.

8. The use of claim 3, wherein said second dosage form comprises at least 10 mg of buprenorphine, 20 mg of buprenorphine, 30 mg of buprenorphine, or 40 mg of buprenorphine.

9. The use of claims 1 - 8, wherein said surgery is a simple surgery.

10. The use of claim 9, wherein said simple surgery is arthroscopic surgery, an excision of a mass, or hernia repair.

11. The use of claims 1 - 8, wherein the surgery is spinal fusion, intrathoracic surgery such as coarctation repair, or pelvic surgery, such as transabdominal hysterectomy.

12. The use of claims 1 - 11, wherein said transdermal administration is produced by a transdermal system selected from the group consisting of a topical gel, a lotion, an ointment, a transmucosal system, a transmucosal device, and an iontophoretic delivery system.

## Patentansprüche

1. Verwendung von Buprenorphin in der Herstellung einer transdermalen Dosierungsform zur Behandlung von postoperativen Schmerzen bei einem Patienten, der eine solche Behandlung benötigt, wobei die Behandlung die Verabreichung der Buprenorphinenthaltenen transdermalen Dosierungsform an den Patienten vor der Operation umfasst.

2. Verwendung gemäß Anspruch 1, wobei die Behandlung die Anwendung der Buprenorphin-enthaltenen transdermalen Dosierungsform 12 - 96 Stunden vor der Operation umfasst.

3. Verwendung gemäß Anspruch 1, wobei die Behandlung ferner die Verabreichung einer zweiten Buprenorphin-enthaltenen transdermalen Dosierungsform für eine zweite, postoperative Dosierungsperiode umfasst, wobei die zweite Dosierungsform dieselbe Dosierung an Buprenorphin oder eine größere Dosierung an Buprenorphin umfasst als die erste Dosierungsform.

4. Verwendung gemäß Anspruch 1, wobei die Behandlung ferner weitere nachfolgende Dosierungsperioden mit nachfolgenden Dosierungsformen für einen gegebenen Zeitraum umfasst, wie sie vom Patienten benötigt wird, um die gewünschte Analgesie zu erreichen.

5. Verwendung gemäß Anspruch 1, wobei die Behandlung ferner eine schnelle Dosiseskalation von Buprenorphinpflastern umfasst, um den gewünschten prä-operativen Dosierungsspiegel zu erreichen, wobei das Verfahren beinhaltet:
(a) Verabreichen einer ersten Buprenorphin-enthaltenden transdermalen Dosierungsform an einem Patienten für eine erste Dosierungsperiode, die nicht länger als 5 Tage ist;
(b) Verabreichung einer zweiten Buprenorphin-enthaltenden transdermalen Dosierungsform an einem Patienten für eine zweite Dosierungsperiode, die nicht länger als 5 Tage ist, wobei die Dosierungsform die gleiche Dosierung an Buprenorphin oder eine größere Dosierung an Buprenorphin umfasst als die erste Dosierungsform; und
(c) Verabreichen einer dritten Buprenorphin-enthaltenden transdermalen Dosierungsform an einem Patienten für eine dritte Dosierungsperiode, wobei die dritte Dosierungsform eine größere Dosierung an Buprenorphin umfasst als die zweite Dosierungsform und die dritte Dosierungsform ist das gewünschte Dosierungsniveau für die postoperative Schmerzkontrolle.

6. Verwendung gemäß Anspruch 5, wobei die Behandlung 4 bis 10 Tage vor der Operation begonnen wird.

7. Verwendung gemäß Anspruch 1, wobei die Dosierungsform mindestens 5 mg Buprenorphin umfasst.

8. Verwendung gemäß Anspruch 3, wobei die zweite Dosierungsform mindestens 10 mg Buprenorphin, 20 mg Buprenorphin, 30 mg Buprenorphin oder 40 mg Buprenorphin umfasst.

9. Verwendung gemäß den Ansprüchen 1-8, wobei die Operation eine einfache Operation ist.

10. Verwendung gemäß Anspruch 9, wobei die einfache Operation eine arthroskopische Operation, ein Entfernen einer Masse oder eine Hernie-Reparatur ist.

11. Verwendung gemäß den Ansprüchen 1-8, wobei die Operation eine Wirbelsäulenversteifung, intrathoraktal Operation wie z.B. Koarktationreparatur oder eine Beckenoperation wie z.B. eine transabdominale Hysterektomie ist.

12. Verwendung gemäß den Ansprüchen 1-11, wobei die transdermale Verabreichung durch ein transdermales System ausgewählt aus der Gruppe bestehend aus einem topischen Gel, einer Lotion, einer Salbe, einem transmukosalen System, einem transmukosalen Gerät und einem iontophoretischen Abgabesystem erzeugt wird.

## Revendications

1. Utilisation de buprénorphine pour la production d'une forme posologique transdermique pour le traitement de la douleur postopératoire chez un patient ayant besoin d'un tel traitement, traitement comprenant l'administration audit patient de ladite forme posologique transdermique contenant de la buprénorphine avant une intervention chirurgicale.

2. Utilisation suivant la revendication 1, dans laquelle le traitement comprend l'application de la forme posologique transdermique contenant de la buprénorphine 12 à 96 heures avant l'intervention chirurgicale.

3. Utilisation suivant la revendication 1, dans laquelle le traitement comprend en outre l'administration d'une deuxième forme posologique transdermique contenant de la buprénorphine pendant une deuxième période d'administration postopératoire, ladite deuxième forme posologique comprenant la même dose de buprénorphine que ladite première forme posologique, ou bien comprenant une dose de buprénorphine supérieure à celle-ci.

4. Utilisation suivant la revendication 1, dans laquelle le traitement comprend en outre des périodes d'administration ultérieures prolongées avec des formes posologiques suivantes pendant une période de temps donnée de la manière requise par le patient pour parvenir à l'analgésie désirée.

5. Utilisation suivant la revendication 1, dans laquelle le traitement comprend en outre une augmentation rapide de dose des timbres de buprénorphine pour parvenir à la valeur de posologie préopératoire désirée, dans laquelle la méthode comprend :
(a) l'administration au patient d'une première forme posologique contenant de la buprénorphine pendant une première période d'administration qui est non supérieure à 5 jours ;
(b) l'administration au patient d'une deuxième forme posologique transdermique contenant de la buprénorphine pendant une deuxième période d'administration qui est non supérieure à 5 jours, la deuxième forme posologique comprenant la même dose de buprénorphine que la première forme posologique, ou bien une dose de buprénorphine supérieure à celle-ci ; et
(c) l'administration au patient d'une troisième forme posologique transdermique de buprénorphine pendant une troisième période d'administration, la troisième forme posologique comprenant une dose de buprénorphine supérieure à celle de la deuxième forme posologique, et la troisième forme posologique correspondant à la valeur de posologie désirée pour la lutte postopératoire contre la douleur.

6. Utilisation suivant la revendication 5, dans laquelle le traitement est commencé 4 à 10 jours avant l'intervention chirurgicale.

7. Utilisation suivant la revendication 1, dans laquelle ladite forme posologique comprend au moins 5 mg de buprénorphine.

8. Utilisation suivant la revendication 3, dans laquelle ladite deuxième forme posologique comprend au moins 10 mg de buprénorphine, 20 mg de buprénorphine, 30 mg de buprénorphine ou 40 mg de buprénorphine.

9. Utilisation suivant la revendication 1 ou 8, dans laquelle ladite intervention chirurgicale est une intervention chirurgicale simple.

10. Utilisation suivant la revendication 9, dans laquelle ladite intervention chirurgicale simple est une intervention chirurgicale arthroscopique, une excision d'une masse ou la réparation d'une hernie.

11. Utilisation suivant les revendications 1 à 8, dans laquelle l'intervention chirurgicale est une fusion rachidienne, une intervention intrathoracique telle qu'une réparation de coarctation, ou une intervention chirurgicale pelvienne telle qu'une hystérectomie transabdominale.

12. Utilisation suivant les revendications 1 à 11, dans laquelle ladite administration transdermique est effectuée par un système transdermique choisi dans le groupe consistant en un gel topique, une lotion, une pommade, un système d'administration à travers une muqueuse, un dispositif d'administration à travers une muqueuse et un système d'administration ionophorétique.
